# EUROPEAN PATENT APPLICATION

(11) **EP 3 563 888 A1**
(43) Date of publication of application: **06.11.2019**
(21) Application number: 19171688.5
(22) Date of filing: 29.04.2019
(51) Int. Cl.: A61M 1/36, B01J 20/22, A61M 1/16

(54) **MULTI-STAGE BLOOD PURIFICATION APPARATUS FOR REMOVAL OF TOXINS**

(30) Priority: 30.04.2018 US 201862664364 P; 08.02.2019 US 201916270884
(71) Applicant: Medtronic, Inc., Minneapolis, MN 55432 (US)
(72) Inventor: Gerber, Martin T., Maple Grove, MN Minnesota 55369 (US)
(74) Representative: Maschio, Antonio

(57) **Abstract**

The present invention relates to a multi-stage blood purification apparatus designed to remove substances from a bodily fluid of a subject. In one aspect, the present invention relates to a sorbent stage containing a sorbent material by which a substance, or substances, is removed from a bodily fluid when in contact with the bodily fluid. In another aspect, the present invention provides a multi-stage blood purification apparatus containing a sorbent stage and a membrane stage. Further, methods of purifying a bodily fluid utilizing apparatuses of the present disclosure are provide.

## Description

### CROSS-REFERENCE TO RELATED APPLICATION

This application claims the benefit of and priority to U.S. Provisional Patent Application No. 62/664,364 filed April 30, 2018, the entire disclosure of which is incorporated by reference herein.

### FIELD OF THE INVENTION

The present invention relates to a multi-stage blood purification apparatus designed to remove substances from a bodily fluid of a subject.

### BACKGROUND OF THE INVENTION

Blood purification processes, such as hemodialysis or hemofiltration, typically utilize a filter or membrane across which fluid and/or waste products may travel, thereby removing them from blood. The blood, with reduced fluid or waste products, is then returned to the patient.

However, certain substances may not be removed by this method. Specific substances that may not be cleared through, e.g., high flux or low flux dialyzers include known hydrophobic, protein-bound, non-polar, uremic toxins including p-cresol and indoxyl sulfate.

Accordingly, there is a strong need for blood purification apparatuses and methods designed to remove uremic toxins and other substances from a bodily fluid of a subject in need thereof.

### SUMMARY OF THE INVENTION

The present invention addresses the need mentioned above by providing blood purification apparatuses and methods for the removal of substances from a bodily fluid of a subject in need thereof.

In one aspect, the present disclosure provides a medical device comprises (i) a housing defining an interior, wherein the interior has a fluid compartment, and (ii) a sorbent material within the fluid compartment, wherein a fluid entering the fluid compartment flows in contact with the sorbent such that a solute of the fluid is removed from the fluid by the sorbent material.

In some embodiments, the housing further comprises a fluid inlet port and a fluid outlet port, wherein the fluid inlet port and fluid outlet port are in fluid communication with the fluid compartment and define a flow path through the housing.

In some embodiments, the fluid inlet port and the fluid outlet port are adapted to be connectable to an extracorporeal circuit. In some embodiments, the fluid inlet port and the fluid outlet port each comprise a luer adapter.

In some embodiments, the sorbent material comprises a solid support having at least binding agent bound thereon, wherein the binding agent is capable of binding to the solute. In other embodiments, the solid support comprises one or more of agarose, cellulose, polyether sulfones, polyamides, polysaccharides, polytetrafluoroethylene, polyesters, polyurethanes, polyvinylidene fluoride, polypropylene, fluorocarbons, poly(tetrafluoroethylene-co-perfluoro(alkyl vinyl ether)), polyethylene, glass, polycarbonates, polyacrylate, polyacrylamide, poly(azolactone), polystyrene, ceramics, and nylon. In some embodiments, the at least binding agent is covalently bound to the solid support. In some embodiments, the binding agent is a lipocalin protein.

In some embodiments, the sorbent material is in the form of beads, membranes, gels, columns, chips, plates, tubes, sheets, fibers, or hollow fibers.

In another aspect, the present disclosure provides a multi-stage blood purification device comprising (i) a membrane stage, wherein the membrane stage comprises a housing defining an interior, wherein the interior comprises a blood flow path having a blood inlet port and a blood outlet port, a dialysate flow path having a dialysate inlet port and a dialysate outlet port, and a semipermeable membrane separating the blood flow path and the dialysate flow path; and (ii) a sorbent stage, wherein the sorbent stage comprises a housing defining an interior, wherein the interior comprises a fluid flow path having a fluid inlet port and a fluid outlet port, wherein the fluid flow path is in fluid communication with the blood flow path of the membrane stage; and a sorbent material within the fluid flow path, wherein a fluid entering the fluid flow path of the sorbent stage flows in contact with the sorbent such that a solute of the fluid is removed from the fluid by the sorbent.

In some embodiments, the fluid inlet port of the sorbent stage is connected in fluid communication with the blood outlet port of the membrane stage. In other embodiments, the fluid outlet port of the sorbent stage is connected in fluid communication with the blood inlet port of the membrane stage.

In some embodiments, the sorbent material comprises a solid support having at least binding agent bound thereon, wherein the binding agent is capable of binding to the solute. In other embodiments, the solid support comprises one or more of agarose, cellulose, polyether sulfones, polyamides, polysaccharides, polytetrafluoroethylene, polyesters, polyurethanes, polyvinylidene fluoride, polypropylene, fluorocarbons, poly(tetrafluoroethylene-co-perfluoro(alkyl vinyl ether)), polyethylene, glass, polycarbonates, polyacrylate, polyacrylamide, poly(azolactone), polystyrene, ceramics, and nylon. In some embodiments, the at least binding agent is covalently bound to the solid support. In some embodiments, the binding agent is a lipocalin protein.

In some embodiments, the sorbent material is in the form of beads, membranes, gels, columns, chips, plates, tubes, sheets, fibers, or hollow fibers.

In yet another aspect, the present disclosure provides a method of purifying a bodily fluid of a subject in need thereof, the method comprising receiving a fluid from a subject at an inlet port of a sorbent stage comprising (i) a housing defining an interior, wherein the interior has a fluid compartment in fluid communication with the inlet port; and (ii) a sorbent within the fluid compartment, passing the fluid through the fluid compartment of the sorbent stage, wherein the fluid passing through the fluid compartment of the sorbent stage flows in contact with the sorbent such that a solute of the fluid is removed from the fluid by the sorbent.

In some embodiments, the fluid is received from a blood outlet port of a membrane stage.

In some embodiments, the method further comprises passing the fluid through an outlet port in fluid communication with the fluid compartment of the sorbent stage to a blood inlet port of a membrane stage.

In some embodiments, the method further comprises returning the fluid to the subject after it has passed fluid compartment of the sorbent stage.

In some embodiments, the fluid is blood, or a fluid component thereof.

The details of one or more embodiments of the invention are set forth in the description below. Other features, objectives, and advantages of the invention will be apparent from the description and from the claims.

### BRIEF DESCRIPTION OF THE FIGURES

The accompanying drawings, which are incorporated herein and constitute part of this specification, illustrate the presently preferred embodiments of the invention, and, together with the general description above and the detailed description given below, serve to explain the features of the invention. In the drawings:
**Figure 1** shows an exemplary sorbent stage according to an embodiment of the present disclosure.
**Figure 2** shows a first exemplary multi-stage blood purification apparatus according to an embodiment of the present disclosure.
**Figure 3** shows a second exemplary multi-stage blood purification apparatus according to an embodiment of the present disclosure.

In the drawings, elements of structure similar to those disclosed or designated with the same reference numeral are followed by the lower case letter, *e*.*g*., "a," "b," or "c."

### DETAILED DESCRIPTION OF THE INVENTION

The present invention will now be described more fully hereinafter. However, many modifications and other embodiments of the present invention set forth herein will come to mind to one skilled in the art to which the invention pertains having the benefit of the teachings presented in the foregoing descriptions. Therefore, it is to be understood that the present invention is not to be limited to the specific embodiments disclosed and that modifications and other embodiments are intended to be included within the scope of the appended claims.

All scientific and technical terms used herein have meanings commonly used in the art unless otherwise specified. The definitions provided herein are to facilitate understanding of certain terms used frequently herein and are not meant to limit the scope of the present disclosure.

As used herein, the singular forms "a", "an", and "the" encompass embodiments having plural referents, unless the content clearly dictates otherwise.

As used herein, a "binding agent" means a molecule, compound, nucleic acid, protein, or other substance which is capable of recognizing and associating with one or more specific target molecules, compounds, nucleic acids, proteins, or other ligands.

As used herein, a "blood purification process," or the like, refers to a process in which blood, or one or more components thereof, such as plasma, is cleaned or purified extracorporeal; i.e., waste products are removed from the blood, or component thereof, and cleaned blood, or component thereof, is returned to the patient. Examples of blood purification processes include ultrafiltration, hemofiltration, hemodialysis, hemodiafiltration, peritoneal dialysis and the like. It will be understood that, during some of these processes, fluid may be removed or introduced to the blood, or components thereof, before it is returned to the patient. Any patient for which a blood purification process is indicated may benefit from the devices, systems and methods described herein.

As used herein, the term "chelating resins" refers to a class of resins that interacts and selectively binds with selected ions and ligands, i.e., the process referred to as chelation.

As used herein, the term "communicate" and "communication" include, but are not limited to, the connection of fluid system elements, either directly or remotely, enabling fluid interface among and between said elements.

As used herein, the term "comprising" includes, but is not limited to, whatever follows the word "comprising." Thus, use of the term indicates that the listed elements are required or mandatory but that other elements are optional and may or may not be present.

As used herein, the term "connectable" refers to being able to be joined together for purposes including, but not limited to, allowing a flow of fluid. The term "connectable" can refer to being able to be joined together temporarily or permanently.

As used herein, the terms "contact," "contacted," or "contacting" denotes (1) a coming together or touching, as of objects or surfaces; (2) the state or condition of touching or of being in immediate proximity; (3) connection or interaction. For example, in reference to a "fluid contacting a sorbent material" refers to fluid that has come together, has touched, or is in immediate proximity to connect or interact with any material or material layer of a sorbent container, system or cartridge.

As used herein, a "contrast agent" is a substance used to enhance the contrast of structures or fluids within the body in medical imaging. Exemplary contrast agents may include, but are not limited to, iodine and barium for enhancing x-ray-based imaging methods or gadolinium for enhancing magnetic resonance imaging.

As used herein, the term "detachable" refers to a characteristic of an object or apparatus that permits it to be removed and/or disconnected from another object or apparatus.

As used herein, the term "dialysate" describes a fluid into or out of which solutes from a fluid to be dialyzed diffuse through a membrane. A dialysate typically contains electrolytes that are close in concentration to the physiological concentration of electrolytes found in blood.

As used herein, "dialysis" is a type of filtration, or a process of selective diffusion through a membrane. Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids. The dialysate is used to remove solutes from the fluid to be dialyzed. The dialysate can also provide enrichment to the other fluid.

As used herein, the terms "dialysis membrane," "hemodialysis membrane," "hemofiltration membrane," "hemodiafiltration membrane," "ultrafiltration membrane," and generally "membrane," refer, in context, to a semi-permeable barrier selective to allow diffusion and convection of solutes of a specific range of molecular weights through the barrier that separates blood and dialysate, or blood and filtrate, while allowing diffusive and/or convective transfer between the blood on one side of the membrane and the dialysate or filtrate circuit on the other side of the membrane.

As used herein, the term "dialyzer" refers to a cartridge or container with two flow paths separated by semi-permeable membranes. One flow path is for blood and one flow path is for dialysate. The membranes can be in the form of hollow fibers, flat sheets, or spiral wound or other conventional forms known to those of skill in the art. Membranes can be selected from the following materials of polysulfone, polyethersulfone, poly(methyl methacrylate), modified cellulose, or other materials known to those skilled in the art.

As used herein, the term "endogenous" means having an internal cause or origin, *i*.*e*., from within a patient or subject. For example, an endogenous substance may be a metabolic waste product such as urea, p-cresol, or indoxyl sulfate.

As used herein, the term "exogenous" means originating from outside an organism. For example, an exogenous component of blood may be a toxin or poison, an administered therapeutic, or any other administered substance such as a contrast agent used for visualization.

As used herein, the term "extracorporeal," as used herein generally means situated or occurring outside the body.

As used herein, the term "extracorporeal circuit" refers to a fluid pathway incorporating one or more components such as, but not limited to, conduits, valves, pumps, fluid connection ports or sensing devices configured therein such that the pathway conveys blood from a subject to an apparatus for hemodialysis, hemofiltration, hemodiafiltration, ultrafiltration, or other blood purification process and back to the subject.

As used herein, the term "flow path" refers to a route or a collection of routes for a fluid to move within. Within a flow path there may be more than one route that a fluid can follow to move from a first position to a second position. A fluid may move through a flow path such that it recirculates, or passes the same position more than once as it moves through a flow path. A flow path may be a single element such as a tube, or a flow path may be a grouping of components of any type that guide the movement of a fluid. The term "flow loop" and "flow path" often may be used interchangeably.

As used herein, "have", "having", "include", "including", "comprise", "comprising" or the like are used in their open ended sense, and generally mean "including, but not limited to."

As used herein, the tem "hemodiafiltration" is a therapy that combines hemofiltration and hemodialysis.

As used herein, the term "hemofiltration" is a therapy in which blood is filtered across a semi-permeable membrane. Water and solutes are removed from the blood via pressure-driven convection across the membrane. The sieving properties of the membrane exclude certain solutes above a certain threshold from crossing the membrane. One common sieving property is "albumin sieving." In most situations it is not desirable to remove albumin during renal replacement therapy, as lower blood serum albumin is associated with increased mortality rates. In hemofiltration, solutes small enough to pass through the membrane in proportion to their plasma concentration are removed. The driving force is a pressure gradient rather than a concentration gradient. A positive hydrostatic pressure drives water and solutes across the filter membrane from the blood compartment to the filtrate compartment, from which it is drained. Solutes, both small and large, get dragged through the membrane at a similar rate by the flow of water that has been engineered by the hydrostatic pressure. Hence, convection overcomes the reduced removal rate of larger solutes (due to their slow speed of diffusion) observed in hemodialysis. The rate of solute removal is proportional to the amount of fluid removed from the blood circuit, which can be adjusted to meet the needs of a clinical situation. In general, the removal of large amounts of plasma water from the patient requires volume substitution. Substitution fluid, typically a buffered solution close to the plasma water composition a patient needs, can be administered pre or post filter (predilution mode, post-dilution mode).

As used herein, "hemodialysis" is a technique where blood and a "cleansing fluid" called dialysate are exposed to each other separated by a semi-permeable membrane. Solutes within the permeability range of the membrane pass while diffusing along existing concentration gradients. Water and solutes are also transferred by convection across a pressure gradient that may exist across the dialysis membrane. The dialysate employed during hemodialysis has soluble ions such as sodium, calcium and potassium ions and is not pure water. The sieving properties of the membrane exclude certain solutes above a certain threshold from crossing the membrane. One common sieving property is "albumin sieving." In most situations it is not desirable to remove albumin during renal replacement therapy, as lower blood serum albumin is associated with increased mortality rates.

The term "hemofilter" refers to an apparatus (or may refer to a filter) used in hemofiltration. A hemofilter apparatus can be connected to an extracorporeal circuit and configured to operate with a semipermeable membrane that separates at least a portion of the fluid volume from blood to produce a filtrate fluid.

As used herein, the term "inlet port" refers to an opening or aperture through which a fluid first passes to enter an object, apparatus or assembly.

As used herein, the term "lipocalin" refers to a family of proteins composed primarily of extracellular ligand-binding proteins with high specificity for small, hydrophobic molecules that share limited regions of sequence homology and a common tertiary structure architecture. These may include, but are not limited to, alpha-1- microglobulin/ bikunin precursor (AMBP), apolipoprotein D (APOD), apolipoprotein M (APOM), complement component 8, gamma polypeptide (C8G), cellular retinoic acid binding protein 1 (CRABP1), cellular retinoic acid binding protein 2 (CRABP2), fatty acid binding protein 1, liver (FABP1), fatty acid binding protein 12 (FABP12), fatty acid binding protein 2, intestinal (FABP2), fatty acid binding protein 3, muscle and heart (mammaryderived growth inhibitor) (FABP3), fatty acid binding protein 4, adipocyte (FABP4), fatty acid binding protein 5 (psoriasisassociated) (FABP5), fatty acid binding protein 6, ileal (FABP6), fatty acid binding protein 7, brain (FABP7), fatty acid binding protein 9, testis (FABP9), lipocalin 1 (LCN1), lipocalin 10 (LCN10), lipocalin 12 (LCN12), lipocalin 15 (LCN15), lipocalin 1 pseudogene 1 (LCN1P1), lipocalin 2 (LCN2), lipocalin 6 (LCN6), lipocalin 8 (LCN8), lipocalin 9 (LCN9), lipocalin-like 1 (LCNLl), odorant binding protein 2A (OBP2A), odorant binding protein 2B (OBP2B), orosomucoid 1 (ORM1), orosomucoid 2 (ORM2), progestagenassociated endometrial protein (PAEP), peripheral myelin protein 2 (PMP2), prostaglandin D2 synthase 21kDa (brain) (PTGDS), retinol binding protein 1, cellular (RBP1), retinol binding protein 2, cellular (RBP2), retinol binding protein 4, plasma (RBP4), retinol binding protein 5, cellular (RBP5), retinol binding protein 7, cellular (RBP7).

As used herein, the terms "luer connector" and "luer adapter" refer to adapters or connectors conforming to International Standards Organization (ISO) standards 594-2.

As used herein, the term "membrane stage" refers an apparatus, or portion of an apparatus, containing a semi-permeable membrane, hemodialysis membrane, hemofiltration membrane, hemodiafiltration membrane, ultrafiltration membrane, or general membrane for use in a blood purification process. A membrane of a membrane stage operates to allow diffusion and convection of solutes of a specific range of molecular weights through the barrier that separates blood and dialysate, or blood and filtrate, while allowing diffusive and/or convective transfer between the blood on one side of the membrane and the dialysate or filtrate on the other side of the membrane. Examples of membrane stages may include, but are not limited to, a dialyzer or a hemofilter.

As used herein, the term "or" is generally employed in its sense including "and/or" unless the content clearly dictates otherwise.

As used herein, the term "outlet port" refers to an opening or aperture through which a fluid passes to exit an object, apparatus or assembly.

As used herein, a "patient" or "subject" is a member of any animal species, preferably a mammalian species, optionally a human. The subject can be an apparently healthy individual, an individual suffering from a disease, or an individual being treated for a disease.

As used herein, a "patient for which a blood purification process is indicated" is a patient that has undergone, is undergoing, or is likely to undergo at least one blood purification process wherein the blood is processed and returned to the patient. In general, such patients are those with deficient renal clearance, such as those suffering from chronic kidney disease, or acute kidney failure. In other instances, such patients may be suffering from poisoning or other acute conditions for which a blood purification process may be warranted.

As used herein, the term "solid support" refers to any solid material having a surface area to which organic molecules can be attached through bond formation or absorbed through electronic or static interactions such as covalent bond or complex formation through a specific functional group.

As used herein, the term "solute" refers to a substance dissolved, suspended, or present in another substance, usually the component of a solution present in the lesser amount.

As used herein, a "sorbent," "sorbent material," or "sorbent medium" is a substance that has the property to collect molecules of another substance by sorption, e.g., by adsorption or absorption. A sorbent medium is a sorbent through, or around, which a substance such as blood or plasma may be passed so that molecules from the substance may be sorbed to the sorbent medium.

As used herein, the term "sorbent stage" refers to an apparatus, or portion of an apparatus, containing one or more sorbent materials for removing specific solutes from a fluid, such as p-cresol, in a blood purification process.

The terms "treating" and "treatment" refer to the management and care of a patient having a pathology or condition by administration of one or more therapy contemplated by the present invention. Treating also includes administering one or more methods of the present invention or using any of the systems, devices or compositions of the present invention in the treatment of a patient. As used herein, "treatment" or "therapy" refers to both therapeutic treatment and prophylactic or preventative measures. "Treating" or "treatment" does not require complete alleviation of signs or symptoms, does not require a cure, and includes protocols having only a marginal or incomplete effect on a patient.

As used herein, the term "uremic toxin" refers to any solute that is retained in the blood due to, directly or indirectly, deficient renal clearance, including urea, creatine, and beta-2-microglobulin. "Uremic toxin" may also refer to non-polar, hydrophobic, and/or protein-bound compounds such as p-cresol or indoxyl sulfate. Other uremic toxins are disclosed in, *e*.*g*., Vanholder et al., "Review on uremic toxins: Classification, concentration, and interindividual variability," Kidney International, vol. 63 (2003), pp. 1934-1943, incorporated by reference in its entirety.

As used herein, the term "waste products" refers to any molecular or ionic species originating from the patient or subject, including metabolic wastes, molecular or ionic species including nitrogen or sulfur atoms, mid-weight uremic wastes and nitrogenous waste. Waste products are kept within a specific homeostasis range by individuals with a healthy renal system.

In one aspect, the present disclosure is directed toward a dialysis system having a sorbent stage that employs one or more sorbent materials to remove desired solutes from the blood, or a fluid component thereof (*e*.*g*., plasma), of a subject. In particular, the sorbent stage may be used to remove certain uremic toxins that may remain in the blood after typical membrane-based blood

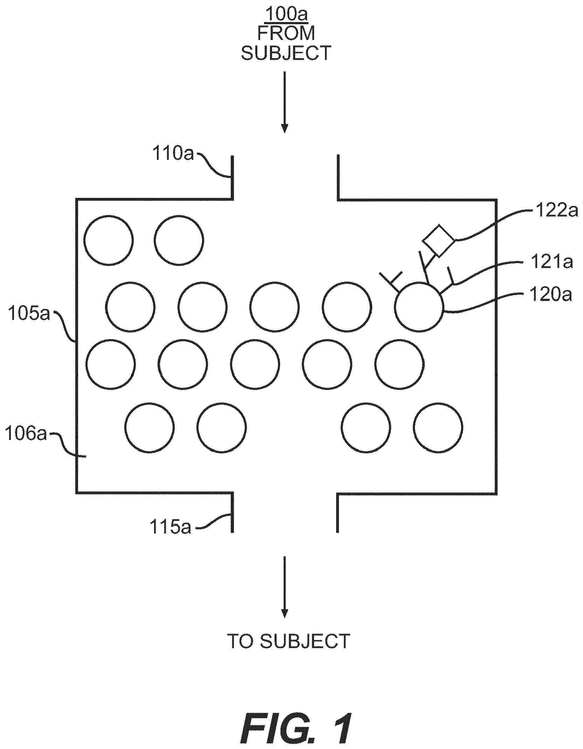

Fig. 1 shows an exemplary sorbent stage **100a** of the present disclosure that may be connected to an extracorporeal circuit during a blood purification process configured to operate via direct contact of the fluid with the sorbent material. The sorbent stage **100a** comprises a housing **105a** defining an interior. The interior has a fluid compartment **106a**. The housing **105a** also comprises a fluid inlet **110a** and a fluid outlet **115a** that are in fluid communication with the fluid compartment **106a**. The fluid inlet **110a** and fluid outlet **115a** may be adapted to allow for the sorbent stage **100a** to be connected to, in a temporary or permanent manner, to an extracorporeal circuit. The fluid inlet **110a** and fluid outlet **115a** may comprise any typical extracorporeal circuit connections, such as standard luer connectors or adapters. Any other suitable means of attachment may be contemplated, including, without limitation, a snap fit, a screw fit, or a glued fit. The sorbent stage **100a** may be placed at any suitable point within an extracorporeal circuit, *e*.*g*., before or after a membrane stage or other blood purification apparatus.

Within the fluid compartment **106a** of the sorbent stage **100a** is a sorbent material **120a.** The sorbent material may be configured to remove one or more endogenous or exogenous substances from the blood of a subject or patient. The sorbent material may be any compatible material and preferably should be non-toxic and stable when exposed to blood or blood components. Endogenous substances may include, by way of non-limiting example, waste products or other solutes, such as non-polar, hydrophobic, and/or protein-bound uremic toxins as known to those of ordinary skill in the art. The substances to be removed may also be exogenous in origin, such as a toxin in a patient with acute or chronic poisoning, or a contrast agent that had been administered for visualization. The sorbent material may include selective binding agents **121a**, such as antibodies, receptors, or other proteins and the like, bound to membranes, fibers, particles, or the like to selectively remove targeted components **122a** from blood. In other embodiments, the sorbent material itself may adsorb or absorb the substance or substances to be removed from the blood, such as a chelating resin or activated charcoal. The sorbent material **120a** may be in any suitable form, *e*.*g*., in the form of beads, membranes, gels, columns, chips, plates, tubes, sheets, fibers, or hollow fibers. The solid support can also be in the form of a coating on the interior of one or more lengths of tubing, piping, or hollow fibers through which a bodily fluid flows. In such embodiments, the tubing, piping, or hollow fibers are preferably coiled or otherwise convoluted or bent, in order to maximize the amount of solid support contacted by the bodily fluid flowing through the tubing, piping, or hollow fibers.

In some embodiments, the sorbent material may comprise a solid support that has at least binding agent bound thereon. In some embodiments, the binding agent may be a lipocalin protein. Lipocalins are a diverse family of proteins composed primarily of extracellular ligand-binding proteins with high specificity for small, hydrophobic molecules. Accordingly, lipocalins may be useful for binding non-polar, hydrophobic, and/or protein-bound uremic toxins. A lipocalin protein for use in an apparatus of the present disclosure may be genetically modified to increase its binding affinity for a particular uremic toxin, and/or improve other binding characteristics as would be understood by those of ordinary skill in the art. Further, the lipocalin protein may be of any origin, e.g., mammalian. In some embodiments, the origin of the lipocalin protein is canine, feline, bovine, caprine, equine, ovine, porcine, rodent, lagomorph, or primate (including human). In other embodiments, the origin of the lipocalin protein is human.

In other embodiments, the solid support may comprise any compound, protein, or substance with an affinity for a component of a bodily fluid for which removal from the fluid is desired. This may include antibodies or other binding proteins. By way of non-limiting example, these antibodies or other binding proteins may have an affinity for albumin. Although in most instances it is not desirable to remove albumin during a blood purification process, albumin is known to bind a variety of small molecules and extend their half-life, including many drugs such as acetaminophen, warfarin, phenobutazone, clofibrate and phenytoin. The toxin ricin is also known to bind to albumin. Accordingly, in one embodiment, apparatuses and methods of the present disclosure may be used to remove albumin-bound drug, molecule, or compound from the blood of a patient or subject in need thereof.

Solid supports for use in the methods described herein preferably should be non-toxic and stable when exposed to blood or blood components. The solid supports may be chosen from among those well known in the art. For example, any suitable porous material may be used as the solid support. Examples of suitable solid supports include, e.g., carbohydrate-based materials such as the various types of SEPHAROSE® (a crosslinked, beaded-form of agarose), e.g., SEPHAROSE 4B®, 4FF®, CL-4B® and CL-6B.

The solid support may be comprised of organic or inorganic molecules, or a combination of organic and inorganic molecules, and may be comprised of one or more functional groups, e.g., hydroxyl groups, suitable for forming covalent bonds with activating agents. The solid support may be comprised of a hydrophilic compound, a hydrophobic compound, or any combination thereof. The solid support may be comprised of a polymer or a copolymer.

Examples of suitable materials for use in solid supports include, but are not limited to, agarose, cellulose, polyether sulfones, polyamides, polysaccharides, polytetrafluoroethylene, polyesters, polyurethanes, polyvinylidene fluoride, polypropylene, fluorocarbons, e.g., poly(tetrafluoroethylene-co-perfluoro(alkyl vinyl ether)), polyethylene, glass, polycarbonates, polyacrylate, polyacrylamide, poly(azolactone), polystyrene, ceramics, and nylon.

The solid support need not be in any particular shape. For example, the solid support may be in the form of beads, membranes, gels, columns, chips, plates, tubes, sheets, fibers, or hollow fibers. The solid support can also be in the form of a coating on the interior of one or more lengths of tubing, piping, or hollow fibers through which a bodily fluid flows. In such embodiments, the tubing, piping, or hollow fibers are preferably coiled or otherwise convoluted or bent, in order to maximize the amount of solid support contacted by the bodily fluid flowing through the tubing, piping, or hollow fibers.

Methods of attaching proteins and ligands to a solid support are well known in the art and may be used to attach a selective binding agent to the solid support of the present disclosure. In some embodiments, the selective binding agent may be covalently coupled to the solid support in a manner that provides more uniform orientation and efficient uremic toxin binding.

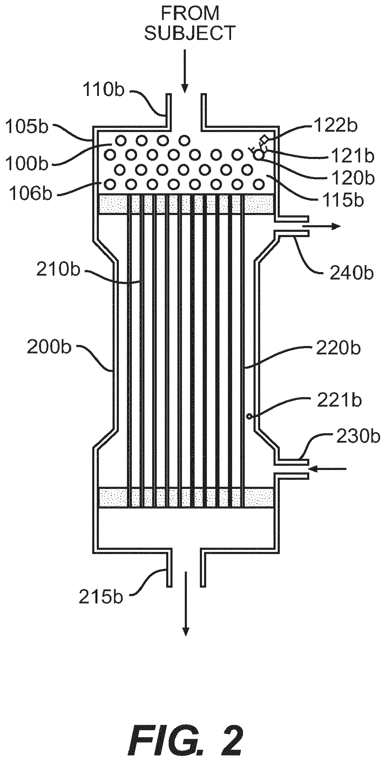

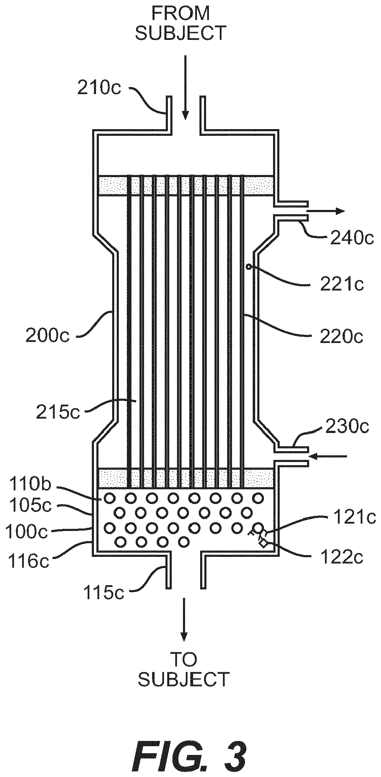

In another aspect of the present disclosure, the sorbent stage may be integrated with a membrane stage, as shown in Figs. 2 and 3. In Fig. 2, sorbent stage **100b** is integrated with membrane stage **200b** to form a multi-stage blood purification apparatus. As in Fig. 1, the sorbent stage **100b** has a housing **105b** defining an interior with a fluid compartment **106b**. The housing further comprises receiving fluid from the terminal ends of membranes 200c, **110b** and a fluid outlet **115b**. The fluid inlet **110b** receives unpurified blood from an extracorporeal circuit and is in fluid communication with the fluid compartment **106b**. Within the fluid compartment **106b** of the sorbent stage **100b** is a sorbent material **120b** as discussed above. In some embodiments, the sorbent material may comprise a selective binding agent **121a** to which targeted components **122b** from blood may bind. Partially cleaned blood then exits the fluid outlet **115b** of the sorbent stage **100b**.

The membrane stage **200b** has a fluid inlet **210b** and a fluid outlet **215b**. In Fig. 2, simplified hollow fiber dialyzer is shown, but any membrane stage useful in a blood purification process may be contemplated, *e*.*g*., a dialyzer utilizing a different format, a hemofiltration apparatus, a hemodiafiltration apparatus, or an ultrafiltration apparatus. The partially cleaned blood exits the fluid outlet **115b** of the sorbent stage **100b** and flows into the membrane stage **200b** through the fluid inlet **210b** of the membrane stage, wherein a potting is generally used to isolate the sorbent stage 100b from the membrane stage 200b. The partially cleaned blood flows into and through hollow fibers **220b** comprising a semi-permeable membrane. The membrane stage further has a dialysate inlet **230b** into which clean dialysate flows. The dialysate flows in a counter current flow to the blood flow, and waste products **221b** are removed from the blood as is understood by those of ordinary skill in the art. The used dialysate flows out of the dialysate outlet **240b**. The cleaned blood flows out of membrane stage outlet **215b** to be returned to the subject or processed further by other components of the extracorporeal circuit.

Fig. 3 illustrates another embodiment of a multi-stage blood purification apparatus in which the blood is first cleaned by a membrane stage **200c**, followed by a sorbent stage **100c**, wherein each stage operates as discussed above. In Fig. 3, the sorbent material **120c** of sorbent stage **100c** is in the form of sheets rather than the beads of Figs. 1 and 2. As discussed above, the sorbent materials **120a, b, or c** may be in any suitable form.

In another aspect, the present disclosure provides methods of purifying a bodily fluid, the method comprising receiving fluid from a patient or subject in need thereof and passing the fluid through an apparatus of the present disclosure. In some embodiments, the patient or subject may be suffering from reduced or non-existent kidney function, *i*.*e*., chronic kidney disease or acute kidney failure. In other embodiments, the patient or subject may be suffering from acute or chronic poisoning. The patient or subject may also be an apparently healthy individual for which a blood purification process is indicated in order to remove a substance from the patient's blood. These may include, but are not limited to, contrast agents or administered therapeutics.

A summary of selected aspects of devices, methods, and systems described herein is provided below.

In a first aspect, a medical device comprises (i) a housing defining an interior, wherein the interior has a fluid compartment, and (ii) a sorbent material within the fluid compartment, wherein a fluid entering the fluid compartment flows in contact with the sorbent such that a solute of the fluid is removed from the fluid by the sorbent material.

A second aspect is a device of the first aspect, wherein the housing further comprises a fluid inlet port and a fluid outlet port, wherein the fluid inlet port and fluid outlet port are in fluid communication with the fluid compartment and define a flow path through the housing.

A third aspect is a device of the second aspect, wherein the fluid inlet port and the fluid outlet port are adapted to be connectable to an extracorporeal circuit.

A fourth aspect is a device of the third aspect, wherein the fluid inlet port and the fluid outlet port each comprise a Luer adapter.

A fifth aspect is a device of the first aspect, wherein the sorbent material comprises a solid support having at least one binding agent bound thereon, wherein the binding agent is capable of binding to the solute.

A sixth aspect is a device of the fifth aspect, wherein the solid support comprises one or more of agarose, cellulose, polyether sulfones, polyamides, polysaccharides, polytetrafluoroethylene, polyesters, polyurethanes, polyvinylidene fluoride, polypropylene, fluorocarbons, e.g., poly(tetrafluoroethylene-co-perfluoro(alkyl vinyl ether)), polyethylene, glass, polycarbonates, polyacrylate, polyacrylamide, poly(azolactone), polystyrene, ceramics, and nylon.

A seventh aspect is a device of the fifth aspect, wherein the at least one binding agent is covalently bound to the solid support.

An eighth aspect is a device of the fifth or seventh aspect, wherein the binding agent is a lipocalin protein.

An ninth aspect is a device of the first aspect, wherein the sorbent material is in the form of beads, membranes, gels, columns, chips, plates, tubes, sheets, fibers, or hollow fibers.

A tenth aspect is a multi-stage blood purification device comprising (i) a membrane stage, wherein the membrane stage comprises a housing defining an interior, wherein the interior comprises a blood flow path having a blood inlet port and a blood outlet port, a dialysate flow path having a dialysate inlet port and a dialysate outlet port, and a semipermeable membrane separating the blood flow path and the dialysate flow path; and (ii) a sorbent stage, wherein the sorbent stage comprises a housing defining an interior, wherein the interior comprises a fluid flow path having a fluid inlet port and a fluid outlet port, wherein the fluid flow path is in fluid communication with the blood flow path of the membrane stage; and a sorbent material within the fluid flow path, wherein a fluid entering the fluid flow path of the sorbent stage flows in contact with the sorbent such that a solute of the fluid is removed from the fluid by the sorbent.

A eleventh aspect is a device of the ninth aspect, wherein the fluid inlet port of the sorbent stage is connected in fluid communication with the blood outlet port of the membrane stage.

An twelfth aspect is a device of the ninth aspect, wherein the fluid outlet port of the sorbent stage is connected in fluid communication with the blood inlet port of the membrane stage.

A thirteenth aspect is a device of the ninth aspect, wherein the sorbent comprises a solid support having at least one binding agent bound thereon, wherein the binding agent is capable of binding to the solute.

A fourteenth aspect is a device of the twelfth aspect, wherein the solid support comprises one or more of agarose, cellulose, polyether sulfones, polyamides, polysaccharides, polytetrafluoroethylene, polyesters, polyurethanes, polyvinylidene fluoride, polypropylene, fluorocarbons, e.g., poly(tetrafluoroethylene-co-perfluoro(alkyl vinyl ether)), polyethylene, glass, polycarbonates, polyacrylate, polyacrylamide, poly(azolactone), polystyrene, ceramics, and nylon.

A fifteenth aspect is a device of the twelfth aspect, wherein the at least one binding agent is covalently bound to the solid support.

A sixteenth aspect is a device of the thirteenth or fifteenth aspect, wherein the binding agent is a lipocalin protein.

A seventeenth aspect is a device of the ninth aspect, wherein the solid support is in the form of beads, membranes, gels, columns, chips, plates, tubes, sheets, fibers, or hollow fibers.

An eighteenth aspect is a method of purifying a bodily fluid of a subject in need thereof, the method comprising receiving a fluid from a subject at an inlet port of a sorbent stage comprising (i) a housing defining an interior, wherein the interior has a fluid compartment in fluid communication with the inlet port; and (ii) a sorbent within the fluid compartment, passing the fluid through the fluid compartment of the sorbent stage, wherein the fluid passing through the fluid compartment of the sorbent stage flows in contact with the sorbent such that a solute of the fluid is removed from the fluid by the sorbent.

A nineteenth aspect is a method of the sixteenth aspect, wherein the fluid is received from a blood outlet port of a membrane stage.

An twentieth aspect is a method of the sixteenth aspect, further comprising passing the fluid through an outlet port in fluid communication with the fluid compartment of the sorbent stage to a blood inlet port of a membrane stage.

A twenty first aspect is a method of the sixteenth aspect, further comprising returning the fluid to the subject after it has passed fluid compartment of the sorbent stage.

A twenty second aspect is a method of the sixteenth aspect, wherein the fluid is blood, or a fluid component thereof.

It will be apparent to one of ordinary skill in the art that pumps, valves, or other components that may be employed in the field of blood purification processes, while not shown, may be used in the devices, systems, and methods described herein to facilitate any aspect of the blood purification process such as to drive the flow of fluid through an extracorporeal circuit, or the like.

It will be further apparent to one of ordinary skill in the art that various combinations and/or modifications and variations can be made in the blood purification systems and methods of the present disclosure depending upon the specific needs for operation and as dictated by the therapeutic needs of the patient. Moreover, features illustrated or described as being part of one embodiment may be used on another embodiment to yield a still further embodiment.

The invention may be described by reference to the following numbered paragraphs:-
1. A medical device comprising:
   a housing defining an interior, wherein the interior has a fluid compartment; and
   a sorbent material within the fluid compartment,
   wherein a fluid entering the fluid compartment flows in contact with the sorbent such that a solute of the fluid is removed from the fluid by the sorbent material.
2. The medical device of paragraph 1, wherein the housing further comprises a fluid inlet port and a fluid outlet port, wherein the fluid inlet port and fluid outlet port are in fluid communication with the fluid compartment and define a flow path through the housing.
3. The medical device of paragraph 2, wherein the fluid inlet port and the fluid outlet port are adapted to be connectable to an extracorporeal circuit.
4. The medical device of paragraph 3, wherein the fluid inlet port and the fluid outlet port each comprise a luer adapter.
5. The medical device of paragraph 1, wherein the sorbent comprises a solid support having at least binding agent bound thereon, wherein the binding agent is capable of binding to the solute.
6. The medical device of paragraph 5, wherein the solid support comprises one or more of agarose, cellulose, polyether sulfones, polyamides, polysaccharides, polytetrafluoroethylene, polyesters, polyurethanes, polyvinylidene fluoride, polypropylene, fluorocarbons, poly(tetrafluoroethylene-co-perfluoro(alkyl vinyl ether)), polyethylene, glass, polycarbonates, polyacrylate, polyacrylamide, poly(azolactone), polystyrene, ceramics, and nylon.
7. The medical device of paragraph 5, wherein the at least one binding agent is covalently bound to the solid support.
8. The medical device of paragraphs 5 or 7, wherein the binding agent is a lipocalin protein.
9. The medical device of paragraph 1, wherein the sorbent material is in the form of beads, membranes, gels, columns, chips, plates, tubes, sheets, fibers, or hollow fibers.
10. A multi-stage blood purification device comprising:
   a. a membrane stage, wherein the membrane stage comprises a housing defining an interior, wherein the interior comprises a blood flow path having a blood inlet port and a blood outlet port, a dialysate flow path having a dialysate inlet port and a dialysate outlet port, and a semipermeable membrane separating the blood flow path and the dialysate flow path; and
      a sorbent stage, wherein the sorbent stage comprises
      a housing defining an interior, wherein the interior comprises a fluid flow path having a fluid inlet port and a fluid outlet port, wherein the fluid flow path is in fluid communication with the blood flow path of the membrane stage; and
      a sorbent material within the fluid flow path,
      wherein a fluid entering the fluid flow path of the sorbent stage flows in contact with the sorbent such that a solute of the fluid is removed from the fluid by the sorbent material.
11. The multi-stage blood purification device of paragraph 10, wherein the fluid inlet port of the sorbent stage is connected in fluid communication with the blood outlet port of the membrane stage.
12. The multi-stage blood purification device of paragraph 10, wherein the fluid outlet port of the sorbent stage is connected in fluid communication with the blood inlet port of the membrane stage.
13. The multi-stage blood purification device of paragraph 10, wherein the sorbent material comprises a solid support having at least one binding agent bound thereon, wherein the binding agent is capable of binding to the solute.
14. The multi-stage blood purification device of paragraph 13, wherein the solid support comprises one or more of agarose, cellulose, polyether sulfones, polyamides, polysaccharides, polytetrafluoroethylene, polyesters, polyurethanes, polyvinylidene fluoride, polypropylene, fluorocarbons, poly(tetrafluoroethylene-co-perfluoro(alkyl vinyl ether)), polyethylene, glass, polycarbonates, polyacrylate, polyacrylamide, poly(azolactone), polystyrene, ceramics, and nylon.
15. The multi-stage blood purification device of paragraph 13, wherein the at least one binding agent is covalently bound to the solid support.
16. The multi-stage blood purification device of paragraph 13 or 15, wherein the binding agent is a lipocalin protein
17. The multi-stage blood purification device of paragraph 10, wherein the solid support is in the form of beads, membranes, gels, columns, chips, plates, tubes, sheets, fibers, or hollow fibers.
18. A method of purifying a bodily fluid of a subject in need thereof, the method comprising:
   receiving a fluid from a subject at an inlet port of a sorbent stage comprising a housing defining an interior, wherein the interior has a fluid
   compartment in fluid communication with the inlet port; and a sorbent within the fluid compartment,
   passing the fluid through the fluid compartment of the sorbent stage, wherein the fluid passing through the fluid compartment of the sorbent stage flows in contact with the sorbent such that a solute of the fluid is removed from the fluid by the sorbent.
19. The method of paragraph 18, wherein the fluid is received from a blood outlet port of a membrane stage.
20. The method of paragraph 18, further comprising passing the fluid through an outlet port in fluid communication with the fluid compartment of the sorbent stage to a blood inlet port of a membrane stage.
21. The method of paragraph 18, further comprising returning the fluid to the subject after it has passed fluid compartment of the sorbent stage.
22. The method of paragraph 18, wherein the fluid is blood, or a fluid component thereof.

## Claims

1. A medical device comprising:
a housing defining an interior, wherein the interior has a fluid compartment; and
a sorbent material within the fluid compartment,
wherein a fluid entering the fluid compartment flows in contact with the sorbent such that a solute of the fluid is removed from the fluid by the sorbent material.

2. The medical device of claim 1, wherein the housing further comprises a fluid inlet port and a fluid outlet port, wherein the fluid inlet port and fluid outlet port are in fluid communication with the fluid compartment and define a flow path through the housing.

3. The medical device of claim 1 or claim 2, wherein the fluid inlet port and the fluid outlet port are adapted to be connectable to an extracorporeal circuit.

4. The medical device of claim 3, wherein the fluid inlet port and the fluid outlet port each comprise a luer adapter.

5. The medical device of any preceding claim, wherein the sorbent comprises a solid support having at least binding agent bound thereon, wherein the binding agent is capable of binding to the solute.

6. The medical device of claim 5, wherein the solid support comprises one or more of agarose, cellulose, polyether sulfones, polyamides, polysaccharides, polytetrafluoroethylene, polyesters, polyurethanes, polyvinylidene fluoride, polypropylene, fluorocarbons, poly(tetrafluoroethylene-co-perfluoro(alkyl vinyl ether)), polyethylene, glass, polycarbonates, polyacrylate, polyacrylamide, poly(azolactone), polystyrene, ceramics, and nylon.

7. The medical device of claim 5 or claim 6, wherein the at least one binding agent is covalently bound to the solid support.

8. The medical device of claim 5 or 7, wherein the binding agent is a lipocalin protein.

9. The medical device of any preceding claim, wherein the sorbent material is in the form of beads, membranes, gels, columns, chips, plates, tubes, sheets, fibers, or hollow fibers.

10. A multi-stage blood purification device comprising:
a. a membrane stage, wherein the membrane stage comprises
a housing defining an interior, wherein the interior comprises a blood flow path having a blood inlet port and a blood outlet port, a dialysate flow path having a dialysate inlet port and a dialysate outlet port, and a semipermeable membrane separating the blood flow path and the dialysate flow path; and
a sorbent stage, wherein the sorbent stage comprises a medical device of any of claims 1 to 9.
